# EUROPEAN PATENT SPECIFICATION

(11) **EP 4 433 155 B1**
(45) Date of publication and mention of the grant of the patent: **01.10.2025**
(21) Application number: 22822867.2
(22) Date of filing: 10.11.2022
(51) Int. Cl.: A61N 1/372

(54) **COMPUTER IMPLEMENTED METHOD AND SYSTEM FOR PROTECTING A PATIENT CRITICAL FIRMWARE FUNCTION OF AN IMPLANTABLE MEDICAL DEVICE**
COMPUTERIMPLEMENTIERTES VERFAHREN UND SYSTEM ZUM SCHÜTZEN EINER PATIENTENKRITISCHEN FIRMWARE-FUNKTION EINER IMPLANTIERBAREN MEDIZINISCHEN VORRICHTUNG
PROCÉDÉ MIS EN UVRE PAR ORDINATEUR ET SYSTÈME DE PROTECTION D'UNE FONCTION MICROLOGICIELLE CRITIQUE POUR LE PATIENT D'UN DISPOSITIF MÉDICAL IMPLANTABLE

(30) Priority: 19.11.2021 EP 21209161
(43) Date of publication of application: 25.09.2024
(73) Proprietor: BIOTRONIK SE & Co. KG, 12359 Berlin (DE)
(72) Inventor: SCHMOLINSKY, Klaus, 13053 Berlin (DE)
(74) Representative: Biotronik Corporate Services SE
(86) International application number: PCT/EP2022/081499
(87) International publication number: WO 2023/088782

(56) References cited:
- EP-A1- 3 785 759
- US-A1- 2001 041 920
- US-A1- 2003 144 711
- US-A1- 2015 293 803
- US-A1- 2017 299 667

## Description

The invention relates to a computer implemented method for protecting a patient critical firmware function of an implantable medical device, in particular a pacemaker, a defibrillator and/or a neuro-stimulator, against unintended execution.

Furthermore, the invention relates to a system for protecting a patient critical firmware function of an implantable medical device, in particular a pacemaker, a defibrillator and/or a neuro-stimulator, against unintended execution.

Patient-critical firmware functions such as Brady_OFF_Mode, Therapy _OFF_State, or similar of implants, e.g., ICDs, S-ICD, IPGs, iLPs, or similar active implants must be protected from unintended execution due to internal firmware errors and/or misuse by cyberattacks.

EP 3 791 925 A1 discloses a leadless pacemaker comprising at least one fixation element for fixating the leadless pacemaker to cardiac tissue, a communication unit which is electrically connected to said fixation element, so that said fixation element is configured to act as a communication antenna for transmitting signals generated by said communication unit to an external device and/or receiving signals from an external device, and a therapy unit for generating electrical signals to electrically stimulate cardiac tissue, wherein said fixation element is configured to act as an electrode for electrically stimulating cardiac tissue and/or sensing electrical signals of the cardiac tissue.

EP 3 785 759 A1 discloses s medical device that is at least partially implantable. The medical device includes an application component configured to apply a therapeutic treatment and/or stimulation signals to a patient. The medical device includes a wireless communication transceiver and a computer memory storing a parameters library and computer readable instructions. A processor is configured to execute the computer readable instructions so as to perform the following steps: control application of therapeutic treatment and/or stimulation signals, via the application component, based on parameters stored in the parameters library; receive, via the wireless communications transceiver, update data representing an update to the parameters library from an external device or system; transmit at least the update data for verification by a blockchain network and addition of the update data to a verified blockchain public ledger including the update data; receive, via the wireless transceiver, blockchain public ledger data based on the verified blockchain public ledger; validate the update to the parameters library based at least on a first condition that the received blockchain public ledger data includes the update data; when the update to the parameters library is validated, control application of therapeutic treatment and/or stimulation signals, via the application component, based on parameters stored in the parameters library including the update data; and when the update to the parameters library is not validated, control the therapy application component so that the update data is not used.

US 2015 029 3803 A1 discloses methods and articles of manufacture for hosting a safety critical application on an uncontrolled data processing device. Various combinations of installation, functional, host integrity, coexistence, interoperability, power management, and environment checks are performed at various times to determine if the safety critical application operates properly on the device. The operation of the SCA on the UDPD may be controlled accordingly.

US 2017 029 9667 A1 discloses a method for operating a magnetic resonance apparatus by a safety unit, taking into account persons fitted with an implant, a safety unit, a safety system, a magnetic resonance apparatus, and a computer program product are provided. The magnetic resonance apparatus includes a first part and a second part. The first part is operated separately from the second part and includes the safety unit. During an examination of a person fitted with an implant, the safety unit checks that the magnetic resonance apparatus, in a restricted operating mode, is complying with implant-conformant limit values.

US 2003 014 4711 A1 discloses an interactive implantable medical device system including an implantable medical device and a network-enabled external device capable of bidirectional communication and interaction with the implantable medical device. The external device is programmed to interact with other similarly-enabled devices. The system facilitates improved patient care by eliminating unnecessary geographic limitations on implantable medical device interrogation and programming, and by allowing patients, physicians, and other users to access medical records, history, and information and to receive status and care-related alerts and messages anywhere there is access to a communications network.

US 2001 004 1920 A1 discloses an implanted medical device (e.g. infusion pump) and handheld communication device wherein the implantable device is capable of operating under control of different software programs, wherein a first program operates after resetting the implantable device and is not capable of providing significant medical functionality but is capable of selected telemetry operations including telemetry operations that allow replacement software to be downloaded, and wherein a second program may be caused to take control of the device and is capable of significant medical functionality and selected telemetry operations but is incapable of receiving replacement software. A software image may be received in multiple messages where each message is provided with its own validation code and wherein a validation code for the whole image is provided and wherein each provided validation code must compared to a derived validation code prior to accepting the validity of the replacement software.

Currently with such implants, certain firmware functions are protected with appropriate checksums to check for potential code modifications e.g. by bitflips before execution and thus prevent their execution. However, no distinction is made between regular firmware functions and the above-mentioned patient-critical firmware functions which should be protected by an additional layer of security.

It is therefore an object of the present invention to provide an improved method for protecting a patient critical firmware function of an implantable medical device against unintended execution.

The object is solved by a computer implemented method for protecting a patient critical firmware function of an implantable medical device having the features of claim 1.

The object is furthermore solved by a system for protecting a patient critical firmware function of an implantable medical device having the features of claim 10.

In addition, the object is solved by a computer program of claim 11 and the computer-readable data carrier of claim 12. Further developments and advantageous embodiments are defined in the dependent claims.

The present invention provides a computer implemented method for protecting a patient critical firmware function of an implantable medical device, in particular a pacemaker, a defibrillator and/or a neuro-stimulator, against unintended execution.

The method comprises receiving a request for execution of a patient critical firmware function of the implantable medical device and verifying that a user associated with the request is authorized to run the patient critical firmware function.

Furthermore, the method comprises, if the user associated with the request is verified as authorized, reading a first checksum from a first memory area of the implantable medical device or providing a first checksum as part of a code area of the patient critical firmware function of the implantable medical device, wherein the first checksum does not match a correct checksum associated with the patient critical firmware function of the implantable medical device.

The method additionally comprises reading a second checksum from a second memory area of the implantable medical device, wherein the second checksum matches the correct checksum associated with the patient critical firmware function of the implantable medical device and writing the second checksum to a third memory area of the implantable medical device from which a checksum is read before execution of the patient critical firmware function of the implantable medical device.

Moreover, the method comprises computing the correct checksum associated with the patient critical firmware function of the implantable medical device and comparing it to the second checksum and, if the second checksum and the correct checksum match, executing the patient critical firmware function of the implantable medical device.

Furthermore, the present invention provides a system for protecting a patient critical firmware function of an implantable medical device, in particular a pacemaker, a defibrillator and/or a neuro-stimulator, against unintended execution.

The system comprises an implantable medical device and a programmer, wherein the implantable medical device is configured to receive a request by the programmer for execution of a patient critical firmware function of the implantable medical device, wherein the implantable medical device is configured to verify that a user associated with the request is authorized to run the patient critical firmware function, wherein the implantable medical device is configured to read a first checksum from a first memory area of the implantable medical device or to provide a first checksum as part of a code area of the patient critical firmware function of the implantable medical device, wherein the first checksum does not match a correct checksum associated with the patient critical firmware function of the implantable medical device.

The implantable medical device is configured to read a second checksum from a second memory area of the implantable medical device, wherein the second checksum matches the correct checksum associated with the patient critical firmware function of the implantable medical device. Furthermore, the implantable medical device is configured to write the second checksum to a third memory area of the implantable medical device from which a checksum is read before execution of the patient critical firmware function of the implantable medical device.

The implantable medical device is configured to compute the correct checksum associated with the patient critical firmware function of the implantable medical device and to compare it to the second checksum, and wherein the implantable medical device is configured to execute the patient critical firmware function of the implantable medical device if the second checksum and the correct checksum match.

Moreover, the present invention provides a computer-readable data carrier containing program code of a computer program for performing the method according to the present invention when the computer program is executed on a computer.

It is an idea of the present invention to provide a patient critical firmware function with a highly secure checksum, which by default is always incorrect.

This reliably prevents the implant firmware from accidentally or intentionally starting a critical firmware function, e.g. programming of OFF mode for IPGs, Therapy_OFF state for ICDs, etc. due to an internal error or cyber attack, e.g. remotely via a programmer or cardiomessenger, said cardiomessenger being an external BIOTRONIK device that forwards messages and/or data sent by the implant to a Home Monitoring Service Center (HMSC) via mobile radio.

E.g. programming of OFF_mode for IPGs, Therapy_OFF state for ICDs etc. hence requires that a checksum check always precedes the execution of these functions.

It is ensured in the code that this checksum is always checked by the firmware immediately before a critical function is executed. If this is not correct, the function is not executed and e.g. an abusive execution attempt is reported to an internal firmware log book, which can be transmitted to BIOTRONIK e.g. during a follow-up or by transmission in a technical HMSC message. Thus, e.g., attempted cyber attacks can be captured by said monitoring.

Through appropriate strong authorization, e.g. password entry on the programmer by the physician or authorized user, the incorrect checksum is always replaced by a correct checksum only immediately before the function is used, thus making the execution of the critical function possible and permissible.

According to an aspect of the invention, the reading of the first checksum from the first memory area of the implantable medical device or the providing of the first checksum as part of a code area of the patient critical firmware function of the implantable medical device, the reading of the second checksum from a second memory area of the implantable medical device, the writing of the second checksum to a third memory area of the implantable medical device from which a checksum is read before execution of the patient critical firmware function of the implantable medical device, the computing of the correct checksum associated with the patient critical firmware function of the implantable medical device, the comparing it to the second checksum and the execution of the patient critical firmware function of the implantable medical device are performed by a non-interruptible compound command. This way, said method steps are a non-interruptible, which results in an additional layer of security.

According to a further aspect of the invention, during execution of the compound command of the patient critical firmware function of the implantable medical device, the second checksum is overwritten by the first checksum, said first checksum being read from a memory buffer or from a code area of the patient critical firmware function of the implantable medical device. By overwriting the second checksum by the first checksum, execution of the patient critical firmware function is no longer enabled.

According to a further aspect of the invention, after overwriting the second checksum by the first checksum, the compound command of the patient critical firmware function of the implantable medical device is terminated. Since the execution of the compound command generally only encompasses transmission of a predefined command to enable or disable specified functions of the implantable medical device, the execution time is short. As soon as the predefined command has been executed the second checksum is thus overwritten by the first checksum thus effectively terminating the compound command.

According to a further aspect of the invention, the second checksum is read from a hardware read-only register of the implantable medical device, and wherein the checksum is a cyclical redundancy check, XOR, modulus or a cryptographic hash, in particular MD5, SHA-1 or SHA-2. This advantageously the provides an effective protection of the patient critical firmware function.

According to a further aspect of the invention, the patient critical firmware function of the implantable medical device is executed by accessing a graphical user interface of a programmer or an app operating on mobile device, in particular a smartphone or tablet device, said programmer or mobile device being configured to communicate wirelessly with the implantable medical device. The implantable medical device can thus be accessed in a plurality of manners.

According to a further aspect of the invention, a user authentication procedure comprises a password input or a two-factor authentication comprising a password input and an additional security feature on the programmer or a web-interface configured to control the programmer, and wherein the user session comprises a session ID and a timestamp. This ensures access to the implantable medical device by only authorized users.

According to a further aspect of the invention, the correct checksum associated with the patient critical firmware function of the implantable medical device is computed and compared to the second checksum within a predefined time span, in particular up to 500ms, prior to execution of the patient critical firmware function of the implantable medical device. By limiting the time span for conducting the checksum computation, an additional layer of security is provided.

According to a further aspect of the invention, the second checksum is written at a factory initialization of the implantable device to a predefined memory cell, said memory cell being accessible by running a predefined register code. Accidental execution of the patient critical firmware function is thus effectively prevented due to the fact that said predefined register code is not part of the code for executing the patient critical firmware function.

The herein described features of the computer implemented method for protecting a patient critical firmware function of an implantable medical device are also disclosed for the system for protecting a patient critical firmware function of an implantable medical device and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a computer implemented method for protecting a patient critical firmware function of an implantable medical device according to a preferred embodiment of the invention; and
- Fig. 2: shows a schematic view of a system for protecting a patient critical firmware function of an implantable medical device according to the preferred embodiment of the invention.

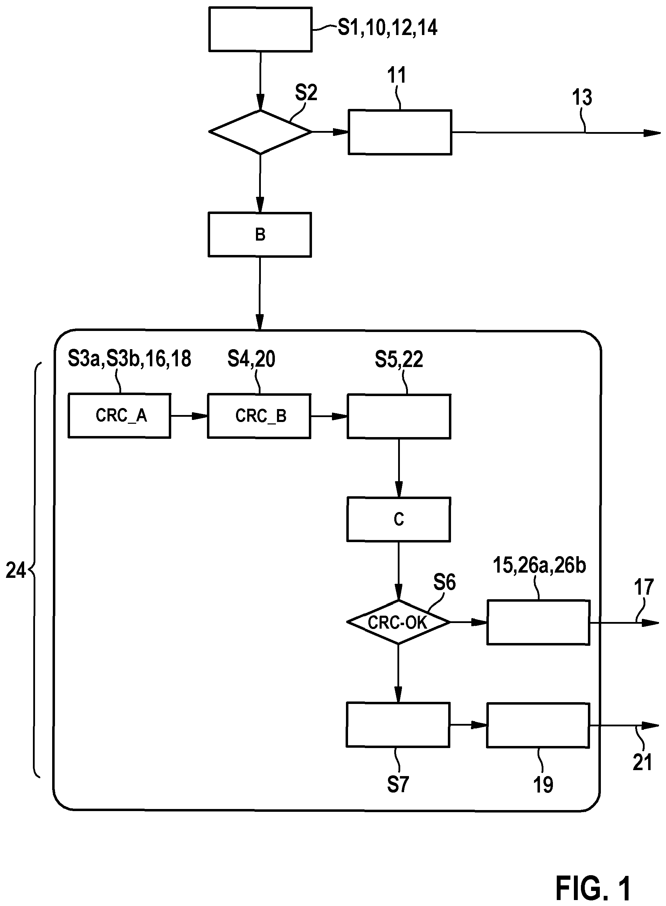

The computer implemented method of Fig. 1 serves to protect a patient critical firmware function 10 of an implantable medical device 12, in particular a pacemaker, a defibrillator and/or a neuro-stimulator, against unintended execution.

The method comprises receiving S1 a request 14 for execution of a patient critical firmware function 10 of the implantable medical device 12 and verifying S2 that a user associated with the request 14 is authorized to run the patient critical firmware function 10.

If the user is confirmed to be authorized, a composite command 24 in step B is started. If the user is not authorized, use of the patient critical firmware function 10 is denied. In addition or optionally an entry in the cyber logbook can be made in step 11. This in turn will cancel the (command) request 14 in step 13.

Furthermore, the method comprises, if the user associated with the request 14 is verified as authorized, reading S3a a first checksum CRC_A from a first memory area 16 of the implantable medical device 12 or providing S3b a first checksum CRC_A as part of a code area 18 of the patient critical firmware function 10 of the implantable medical device 12. The first checksum CRC_A does not match a correct checksum CRC_OK associated with the patient critical firmware function 10 of the implantable medical device 12.

The method additionally comprises reading S4 a second checksum CRC_B from a second memory area 20 of the implantable medical device 12, wherein the second checksum CRC_B matches the correct checksum CRC_OK associated with the patient critical firmware function 10 of the implantable medical device 12.

Moreover, the method comprises writing S5 the second checksum CRC_B to a third memory area 22 of the implantable medical device 12 from which a checksum is read before execution of the patient critical firmware function 10 of the implantable medical device 12. Subsequently, patient critical firmware function 10 is started in step C.

Furthermore, the method comprises computing S6 the correct checksum CRC_OK associated with the patient critical firmware function 10 of the implantable medical device 12 and comparing it to the second checksum CRC_B. If the second checksum CRC_B and the correct checksum CRC_OK match, executing S7 the patient critical firmware function 10 of the implantable medical device 12.

If the second checksum CRC_B and the correct checksum CRC_OK do not match, use of the patient critical firmware function is denied and an entry in the error log is made in step 15. Additionally, the composite command is canceled in step 17.

The steps the reading S3a, S3b, S4, S5, S6 and S7 of the patient critical firmware function 10 of the implantable medical device 12 are performed by a non-interruptible compound command 24.

During execution of the compound command 24 of the patient critical firmware function 10 of the implantable medical device 12, the second checksum CRC_B is overwritten in step 19 by the first checksum CRC_A, said first checksum CRC_A being read from a memory buffer 26a or from a further code area 26b of the patient critical firmware function 10 of the implantable medical device 12.

After overwriting the second checksum CRC_B by the first checksum CRC_A in step 19, the compound command 24 of the patient critical firmware function 10 of the implantable medical device 12 is terminated in step 21.

The second checksum CRC_B is read from a hardware read-only register of the implantable medical device 12, and wherein the second checksum CRC_B is a cyclical redundancy check, XOR, modulus or a cryptographic hash, in particular MD5, SHA-1 or SHA-2.

The patient critical firmware function 10 of the implantable medical device 12 is executed by accessing a graphical user interface of a programmer 30 or an app operating on mobile device 32, in particular a smartphone or tablet device, said programmer 30 or mobile device 32 being configured to communicate wirelessly with the implantable medical device 12.

A user authentication procedure comprises a password input or a two-factor authentication comprising a password input and an additional security feature on the programmer 30 or a web-interface configured to control the programmer 30, and wherein the user session comprises a session ID and a timestamp.

The correct checksum CRC_OK associated with the patient critical firmware function 10 of the implantable medical device 12 is computed and compared to the second checksum CRC_B within a predefined time span, in particular up to 500ms, prior to execution of the patient critical firmware function 10 of the implantable medical device 12.

The second checksum CRC_B is written at a factory initialization of the implantable medical device 12 to a predefined memory cell, said memory cell being accessible by running a predefined register code.

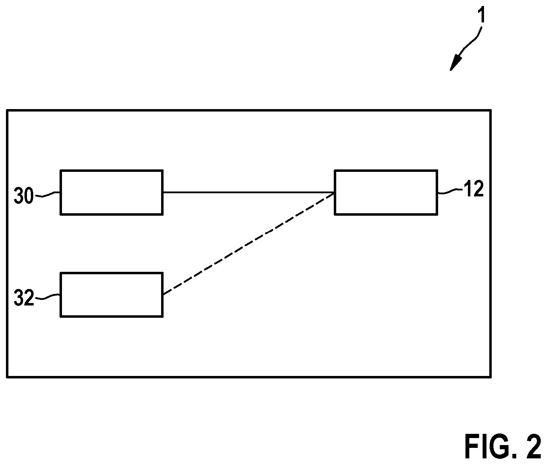

Fig. 2 shows a schematic view of a system for protecting a patient critical firmware function of an implantable medical device according to the preferred embodiment of the invention.

The system 1 comprises an implantable medical device 12 and a programmer 30. Alternatively, the implantable medical device 12 may be controlled by a mobile device 32 being configured to communicate wirelessly with the implantable medical device 12.

The implantable medical device 12 is configured to receive a request 14 by the programmer 30 for execution of a patient critical firmware function 10 of the implantable medical device 12, wherein the implantable medical device 12 is configured to verify that a user associated with the request 14 is authorized to run the patient critical firmware function 10.

The implantable medical device 12 is configured to read a first checksum CRC_A from a first memory area 16 of the implantable medical device 12 or to provide a first checksum CRC_A as part of a code area 18 of the patient critical firmware function 10 of the implantable medical device 12, wherein the first checksum CRC_A does not match a correct checksum CRC_OK associated with the patient critical firmware function 10 of the implantable medical device 12.

Furthermore, the implantable medical device 12 is configured to read a second checksum CRC_B from a second memory area 20 of the implantable medical device 12, wherein the second checksum CRC_B matches the correct checksum CRC_OK associated with the patient critical firmware function 10 of the implantable medical device 12.

Moreover, the implantable medical device 12 is configured to write the second checksum CRC_B to a third memory area 22 of the implantable medical device 12 from which a checksum is read before execution of the patient critical firmware function 10 of the implantable medical device 12.

The implantable medical device 12 is further configured to compute the correct checksum CRC_OK associated with the patient critical firmware function 10 of the implantable medical device 12 and to compare it to the second checksum CRC_B. The implantable medical device 12 is configured to execute the patient critical firmware function 10 of the implantable medical device 12 if the second checksum CRC_B and the correct checksum CRC_OK match.

### Reference Signs

- 1: system
- 10: patient critical firmware function
- 11: method step
- 12: implantable medical device
- 13: method step
- 14: request
- 15: method step
- 16: first memory area
- 17: method step
- 18: code area
- 19: method step
- 20: second memory area
- 21: method step
- 22: third memory area
- 24: compound command
- 26a: memory buffer
- 26b: further code area
- 30: programmer
- 32: mobile device
- B: method step
- C: method step
- CRC_A: first checksum
- CRC_B: second checksum
- CRC_OK: correct checksum
- S1-S7: method steps

## Claims

1. Computer implemented method for protecting a patient critical firmware function (10) of an implantable medical device (12), in particular a pacemaker, a defibrillator and/or a neuro-stimulator, against unintended execution, the method comprising the steps of:
receiving (S1) a request (14) for execution of a patient critical firmware function (10) of the implantable medical device (12);
verifying (S2) that a user associated with the request (14) is authorized to run the patient critical firmware function (10);
if the user associated with the request (14) is verified as authorized, reading (S3a) a first checksum (CRC_A) from a first memory area (16) of the implantable medical device (12) or providing (S3b) a first checksum (CRC_A) as part of a code area (18) of the patient critical firmware function (10) of the implantable medical device (12), wherein the first checksum (CRC_A) does not match a correct checksum (CRC_OK) associated with the patient critical firmware function (10) of the implantable medical device (12);
reading (S4) a second checksum (CRC_B) from a second memory area (20) of the implantable medical device (12), wherein the second checksum (CRC_B) matches the correct checksum (CRC_OK) associated with the patient critical firmware function (10) of the implantable medical device (12);
writing (S5) the second checksum (CRC_B) to a third memory area (22) of the implantable medical device (12) from which a checksum is read before execution of the patient critical firmware function (10) of the implantable medical device (12);
computing (S6) the correct checksum (CRC_OK) associated with the patient critical firmware function (10) of the implantable medical device (12) and comparing it to the second checksum (CRC_B); and
if the second checksum (CRC_B) and the correct checksum (CRC_OK) match, executing (S7) the patient critical firmware function (10) of the implantable medical device (12).

2. Computer implemented method of claim 1, wherein the reading (S3a) of the first checksum (CRC_A) from the first memory area (16) of the implantable medical device (12) or the providing (S3b) of the first checksum (CRC_A) as part of the code area (18) of the patient critical firmware function (10) of the implantable medical device (12), the reading (S4) of the second checksum (CRC_B) from the second memory area (20) of the implantable medical device (12), the writing (S5) of the second checksum (CRC_B) to the third memory area (22) of the implantable medical device (12) from which the checksum is read before execution of the patient critical firmware function (10) of the implantable medical device (12), the computing (S6) of the correct checksum (CRC_OK) associated with the patient critical firmware function (10) of the implantable medical device (12), the comparing it to the second checksum (CRC_B) and the execution (S7) of the patient critical firmware function (10) of the implantable medical device (12) are performed by a non-interruptible compound command (24).

3. Computer implemented method of claim 2, wherein during execution of the compound command (24) of the patient critical firmware function (10) of the implantable medical device (12), the second checksum (CRC_B) is overwritten by the first checksum (CRC_A), said first checksum (CRC_A) being read from a memory buffer (26a) or from a further code area (26b) of the patient critical firmware function (10) of the implantable medical device (12).

4. Computer implemented method of claim 3, wherein after overwriting the second checksum (CRC_B) by the first checksum (CRC_A), the compound command (24) of the patient critical firmware function (10) of the implantable medical device (12) is terminated.

5. Computer implemented method of any one of the preceding claims, wherein the second checksum (CRC_B) is read from a hardware read-only register of the implantable medical device (12), and wherein the second checksum (CRC_B) is a cyclical redundancy check, XOR, modulus or a cryptographic hash, in particular MD5, SHA-1 or SHA-2.

6. Computer implemented method of any one of the preceding claims, wherein the patient critical firmware function (10) of the implantable medical device (12) is executed by accessing a graphical user interface of a programmer (30) or an app operating on mobile device (32), in particular a smartphone or tablet device, said programmer (30) or mobile device (32) being configured to communicate wirelessly with the implantable medical device (12).

7. Computer implemented method of claim 6, wherein a user authentication procedure comprises a password input or a two-factor authentication comprising a password input and an additional security feature on the programmer (30) or a web-interface configured to control the programmer (30), and wherein the user session comprises a session ID and a timestamp.

8. Computer implemented method of any one of the preceding claims, wherein the correct checksum (CRC_OK) associated with the patient critical firmware function (10) of the implantable medical device (12) is computed and compared to the second checksum (CRC_B) within a predefined time span, in particular up to 500ms, prior to execution of the patient critical firmware function (10) of the implantable medical device (12).

9. Computer implemented method of any one of the preceding claims, wherein the second checksum (CRC_B) is written at a factory initialization of the implantable medical device (12) to a predefined memory cell, said memory cell being accessible by running a predefined register code.

10. System (1) for protecting a patient critical firmware function (10) of an implantable medical device (12), in particular a pacemaker, a defibrillator and/or a neuro-stimulator, against unintended execution, the system comprising:
an implantable medical device (12) and a programmer (30), wherein the implantable medical device (12) is configured to receive a request (14) by the programmer (30) for execution of a patient critical firmware function (10) of the implantable medical device (12), wherein the implantable medical device (12) is configured to verify that a user associated with the request (14) is authorized to run the patient critical firmware function (10), wherein the implantable medical device (12) is configured to read a first checksum (CRC_A) from a first memory area (16) of the implantable medical device (12) or to provide a first checksum (CRC_A) as part of a code area (18) of the patient critical firmware function (10) of the implantable medical device (12), wherein the first checksum (CRC_A) does not match a correct checksum (CRC_OK) associated with the patient critical firmware function (10) of the implantable medical device (12), wherein the implantable medical device (12) is configured to read a second checksum (CRC_B) from a second memory area (20) of the implantable medical device (12), wherein the second checksum (CRC_B) matches the correct checksum (CRC_OK) associated with the patient critical firmware function (10) of the implantable medical device (12), wherein the implantable medical device (12) is configured to write the second checksum (CRC_B) to a third memory area (22) of the implantable medical device (12) from which a checksum is read before execution of the patient critical firmware function (10) of the implantable medical device (12), wherein the implantable medical device (12) is configured to compute the correct checksum (CRC_OK) associated with the patient critical firmware function (10) of the implantable medical device (12) and to compare it to the second checksum (CRC_B), and wherein the implantable medical device (12) is configured to execute the patient critical firmware function (10) of the implantable medical device (12) if the second checksum (CRC_B) and the correct checksum (CRC_OK) match.

11. Computer program with program code to perform the method of any one of claims 1 to 9 when the computer program is executed on a computer.

12. Computer-readable data carrier containing program code of a computer program for performing the method of to any one of claims 1 to 9 when the computer program is executed on a computer.

## Patentansprüche

1. Computerimplementiertes Verfahren zum Schützen einer patientenkritischen Firmware-Funktion (10) einer implantierbaren medizinischen Vorrichtung (12), insbesondere eines Herzschrittmachers, eines Defibrillators und/oder eines Neurostimulators, vor unbeabsichtigter Ausführung, wobei das Verfahren die folgenden Schritte umfasst:
Empfangen (S1) einer Anforderung (14) zur Ausführung einer patientenkritischen Firmware-Funktion (10) der implantierbaren medizinischen Vorrichtung (12);
Verifizieren (S2), dass ein Benutzer, der dieser Anforderung (14) zugeordnet ist, autorisiert ist, um die patientenkritische Firmware-Funktion (10) auszuführen;
wenn der Benutzer, der der Anforderung (14) zugeordnet ist, als autorisiert verifiziert ist, Lesen (S3a) einer ersten Prüfsumme (CRC_A) aus einem ersten Speicherbereich (16) der implantierbaren medizinischen Vorrichtung (12) oder Bereitstellen (S3b) einer ersten Prüfsumme (CRC_A) als Teil eines Codebereichs (18) der patientenkritischen Firmware-Funktion (10) der implantierbaren medizinischen Vorrichtung (12), wobei die erste Prüfsumme (CRC_A) nicht mit einer korrekten Prüfsumme (CRC_OK), die der patientenkritischen Firmware-Funktion (10) der implantierbaren medizinischen Vorrichtung (12) zugeordnet ist, übereinstimmt;
Lesen (S4) einer zweiten Prüfsumme (CRC_B) aus einem zweiten Speicherbereich (20) der implantierbaren medizinischen Vorrichtung (12), wobei die zweite Prüfsumme (CRC_B) mit der korrekten Prüfsumme (CRC_OK), die der patientenkritischen Firmware-Funktion (10) der implantierbaren medizinischen Vorrichtung (12) zugeordnet ist, übereinstimmt;
Schreiben (S5) der zweiten Prüfsumme (CRC_B) in einen dritten Speicherbereich (22) der implantierbaren medizinischen Vorrichtung (12), aus dem eine Prüfsumme vor der Ausführung der patientenkritischen Firmware-Funktion (10) der implantierbaren medizinischen Vorrichtung (12) gelesen wurde;
Berechnen (S6) der korrekten Prüfsumme (CRC_OK), die der patientenkritischen Firmware-Funktion (10) der implantierbaren medizinischen Vorrichtung (12) zugeordnet ist, und Vergleichen dieser mit der zweiten Prüfsumme (CRC_B); und
wenn die zweite Prüfsumme (CRC_B) und die korrekte Prüfsumme (CRC_OK) übereinstimmen, Ausführen (S7) der patientenkritischen Firmware-Funktion (10) der implantierbaren medizinischen Vorrichtung (12).

2. Computerimplementiertes Verfahren nach Anspruch 1, wobei das Lesen (S3a) der ersten Prüfsumme (CRC_A) aus dem ersten Speicherbereich (16) der implantierbaren medizinischen Vorrichtung (12) oder das Bereitstellen (S3b) der ersten Prüfsumme (CRC_A) als Teil des Codebereichs (18) der patientenkritischen Firmware-Funktion (10) der implantierbaren medizinischen Vorrichtung (12), das Lesen (S4) der zweiten Prüfsumme (CRC_B) aus dem zweiten Speicherbereich (20) der implantierbaren medizinischen Vorrichtung (12), das Schreiben (S5) der zweiten Prüfsumme (CRC_B) in den dritten Speicherbereich (22) der implantierbaren medizinischen Vorrichtung (12), aus dem die Prüfsumme vor der Ausführung der patientenkritischen Firmware-Funktion (10) der implantierbaren medizinischen Vorrichtung (12) gelesen wurde, das Berechnen (S6) der korrekten Prüfsumme (CRC_OK), die der patientenkritischen Firmware-Funktion (10) der implantierbaren medizinischen Vorrichtung (12) zugeordnet ist, das Vergleichen dieser mit der zweiten Prüfsumme (CRC_B) und die Ausführung (S7) der patientenkritischen Firmware-Funktion (10) der implantierbaren medizinischen Vorrichtung (12) von einem nicht unterbrechbaren Verbundbefehl (24) durchgeführt werden.

3. Computerimplementiertes Verfahren nach Anspruch 2, wobei während der Ausführung des Verbundbefehls (24) der patientenkritischen Firmware-Funktion (10) der implantierbaren medizinischen Vorrichtung (12) die zweite Prüfsumme (CRC_B) von der ersten Prüfsumme (CRC_A) überschrieben wird, wobei die erste Prüfsumme (CRC_A) aus einem Speicherpuffer (26a) oder aus einem weiteren Codebereich (26b) der patientenkritischen Firmware-Funktion (10) der implantierbaren medizinischen Vorrichtung (12) gelesen wird.

4. Computerimplementiertes Verfahren nach Anspruch 3, wobei nach dem Überschreiben der zweiten Prüfsumme (CRC_B) durch die erste Prüfsumme (CRC_A) der Verbundbefehl (24) der patientenkritischen Firmware-Funktion (10) der implantierbaren medizinischen Vorrichtung (12) beendet wird.

5. Computerimplementiertes Verfahren nach einem der vorhergehenden Ansprüche, wobei die zweite Prüfsumme (CRC_B) aus einem schreibgeschützten Hardware-Register der implantierbaren medizinischen Vorrichtung (12) gelesen wird und wobei die zweite Prüfsumme (CRC_B) ein Modul einer zyklischen Redundanzprüfung, XOR, oder ein kryptographischer Hash, insbesondere MD5, SHA-1 oder SHA-2, ist.

6. Computerimplementiertes Verfahren nach einem der vorhergehenden Ansprüche, wobei die patientenkritische Firmware-Funktion (10) der implantierbaren medizinischen Vorrichtung (12) durch Zugreifen auf eine graphische Benutzeroberfläche einer Programmiereinheit (30) oder einer App, die auf einer mobilen Vorrichtung (32), insbesondere einem Smartphone oder einer Tablet-Vorrichtung, läuft, ausgeführt wird, wobei die Programmiereinheit (30) oder die mobile Vorrichtung (32) dazu konfiguriert ist, drahtlos mit der implantierbaren medizinischen Vorrichtung (12) zu kommunizieren.

7. Computerimplementiertes Verfahren nach Anspruch 6, wobei ein Benutzerauthentifizierungsvorgang eine Passworteingabe oder eine Zweifaktor-Authentifizierung, die eine Passworteingabe und ein zusätzliches Sicherheitsmerkmal an der Programmiereinheit (30) oder einer Web-Schnittstelle umfasst, die dazu konfiguriert ist, die Programmiereinheit (30) zu steuern, umfasst, und wobei die Benutzersitzung eine Sitzungs-ID und einen Zeitstempel umfasst.

8. Computerimplementiertes Verfahren nach einem der vorhergehenden Ansprüche, wobei die korrekte Prüfsumme (CRC_OK), die der patientenkritischen Firmware-Funktion (10) der implantierbaren medizinischen Vorrichtung (12) zugeordnet ist, berechnet und mit der zweiten Prüfsumme (CRC_B) innerhalb einer vordefinierten Zeitspanne, insbesondere bis zu 500 ms, vor der Ausführung der patientenkritischen Firmware-Funktion (10) der implantierbaren medizinischen Vorrichtung (12) verglichen wird.

9. Computerimplementiertes Verfahren nach einem der vorhergehenden Ansprüche, wobei die zweite Prüfsumme (CRC_B) bei einer Betriebsinitialisierung der implantierbaren medizinischen Vorrichtung (12) in eine vordefinierte Speicherzelle geschrieben wird, wobei die Speicherzelle durch Ausführen eines vordefinierten Registercodes zugänglich ist.

10. System (1) zum Schützen einer patientenkritischen Firmware-Funktion (10) einer implantierbaren medizinischen Vorrichtung (12), insbesondere eines Herzschrittmachers, eines Defibrillators und/oder eines Neurostimulators, vor unbeabsichtigter Ausführung, wobei das System Folgendes umfasst:
eine implantierbare medizinische Vorrichtung (12) und eine Programmiereinheit (30), wobei die implantierbare medizinische Vorrichtung (12) dazu konfiguriert ist, eine Anforderung (14) von der Programmiereinheit (30) zur Ausführung einer patientenkritischen Firmware-Funktion (10) der implantierbaren medizinischen Vorrichtung (12) zu empfangen, wobei die implantierbare medizinische Vorrichtung (12) dazu konfiguriert ist, zu verifizieren, dass ein Benutzer, der der Anforderung (14) zugeordnet ist, autorisiert ist, die patientenkritische Firmware-Funktion (10) auszuführen, wobei die implantierbare medizinische Vorrichtung (12) dazu konfiguriert ist, eine erste Prüfsumme (CRC_A) aus einem ersten Speicherbereich (16) der implantierbaren medizinischen Vorrichtung (12) zu lesen oder eine erste Prüfsumme (CRC_A) als Teil eines Codebereichs (18) der patientenkritischen Firmware-Funktion (10) der implantierbaren medizinischen Vorrichtung (12) bereitzustellen, wobei die erste Prüfsumme (CRC_A) nicht mit einer korrekten Prüfsumme (CRC_OK), die der patientenkritischen Firmware-Funktion (10) der implantierbaren medizinischen Vorrichtung (12) zugeordnet ist, übereinstimmt, wobei die implantierbare medizinische Vorrichtung (12) dazu konfiguriert ist, eine zweite Prüfsumme (CRC_B) aus einem zweiten Speicherbereich (20) der implantierbaren medizinischen Vorrichtung (12) zu lesen, wobei die zweite Prüfsumme (CRC_B) mit der korrekten Prüfsumme (CRC_OK), die der patientenkritischen Firmware-Funktion (10) der implantierbaren medizinischen Vorrichtung (12) zugeordnet ist, übereinstimmt, wobei die implantierbare medizinische Vorrichtung (12) dazu konfiguriert ist, die zweite Prüfsumme (CRC_B) in einen dritten Speicherbereich (22) der implantierbaren medizinischen Vorrichtung (12) zu schreiben, aus dem eine Prüfsumme vor der Ausführung der patientenkritischen Firmware-Funktion (10) der implantierbaren medizinischen Vorrichtung (12) gelesen wurde, wobei die implantierbare medizinische Vorrichtung (12) dazu konfiguriert ist, die korrekte Prüfsumme (CRC_OK), die der patientenkritischen Firmware-Funktion (10) der implantierbaren medizinischen Vorrichtung (12) zugeordnet ist, zu berechnen und sie mit der zweiten Prüfsumme (CRC_B) zu vergleichen, und wobei die implantierbare medizinische Vorrichtung (12) dazu konfiguriert ist, die patientenkritische Firmware-Funktion (10) der implantierbaren medizinischen Vorrichtung (12) auszuführen, wenn die zweite Prüfsumme (CRC_B) und die korrekte Prüfsumme (CRC_OK) übereinstimmen.

11. Computerprogramm mit Programmcode zum Durchführen des Verfahrens nach einem der Ansprüche 1 bis 9, wenn das Computerprogramm auf einem Computer ausgeführt wird.

12. Computerlesbarer Datenträger, der Programmcode eines Computerprogramms zum Durchführen des Verfahrens nach einem der Ansprüche 1 bis 9 enthält, wenn das Computerprogramm auf einem Computer ausgeführt wird.

## Revendications

1. Procédé mis en œuvre par ordinateur destiné à protéger une fonction de micrologiciel critique pour le patient (10) d'un dispositif médical implantable (12), en particulier d'un stimulateur cardiaque, d'un défibrillateur et/ou d'un neurostimulateur, contre une exécution non souhaitée, le procédé comprenant les étapes consistant à :
recevoir (S1) une requête (14) pour exécution d'une fonction de micrologiciel critique pour le patient (10) du dispositif médical implantable (12) ;
vérifier (S2) qu'un utilisateur associé à la requête (14) est autorisé à exécuter la fonction de micrologiciel critique pour le patient (10) ;
si l'utilisateur associé à la requête (14) est vérifié comme autorisé, lire (S3a) une première somme de contrôle (CRC_A) d'une première zone de mémoire (16) du dispositif médical implantable (12) ou fournir (S3b) une première somme de contrôle (CRC_A) en tant que partie d'une zone de code (18) de la fonction de micrologiciel critique pour le patient (10) du dispositif médical implantable (12), dans lequel la première somme de contrôle (CRC_A) ne correspond pas à une somme de contrôle correcte (CRC_OK) associée à la fonction de micrologiciel critique pour le patient (10) du dispositif médical implantable (12) ;
lire (S4) une seconde somme de contrôle (CRC_B) d'une deuxième zone de mémoire (20) du dispositif médical implantable (12), dans lequel la seconde somme de contrôle (CRC_B) correspond à la somme de contrôle correcte (CRC_OK) associée à la fonction de micrologiciel critique pour le patient (10) du dispositif médical implantable (12) ;
écrire (S5) la seconde somme de contrôle (CRC_B) dans une troisième zone de mémoire (22) du dispositif médical implantable (12) depuis lequel une somme de contrôle est lue avant exécution de la fonction de micrologiciel critique pour le patient (10) du dispositif médical implantable (12) ; calculer (S6) la somme de contrôle correcte (CRC_OK) associée à la fonction de micrologiciel critique pour le patient (10) du dispositif médical implantable (12) et la comparer à la seconde somme de contrôle (CRC_B) ; et
si la seconde somme de contrôle (CRC_B) et la somme de contrôle correcte (CRC_OK) correspondent, exécuter (S7) la fonction de micrologiciel critique pour le patient (10) du dispositif médical implantable (12).

2. Procédé mis en œuvre par ordinateur selon la revendication 1, dans lequel l'étape consistant à lire (S3a) la première somme de contrôle (CRC_A) de la première zone de mémoire (16) du dispositif médical implantable (12) ou l'étape consistant à fournir (S3b) la première somme de contrôle (CRC_A) en tant que partie de la zone de code (18) de la fonction de micrologiciel critique pour le patient (10) du dispositif médical implantable (12), l'étape consistant à lire (S4) la seconde somme de contrôle (CRC_B) de la deuxième zone de mémoire (20) du dispositif médical implantable (12), l'étape consistant à écrire (S5) la seconde somme de contrôle (CRC_B) dans la troisième zone de mémoire (22) du dispositif médical implantable (12) depuis lequel la somme de contrôle est lue avant exécution de la fonction de micrologiciel critique pour le patient (10) du dispositif médical implantable (12), l'étape consistant à calculer (S6) la somme de contrôle correcte (CRC_OK) associée à la fonction de micrologiciel critique pour le patient (10) du dispositif médical implantable (12), l'étape consistant à la comparer à la seconde somme de contrôle (CRC_B) et l'exécution (S7) de la fonction de micrologiciel critique pour le patient (10) du dispositif médical implantable (12) sont réalisées par un ordre composé impossible à interrompre (24).

3. Procédé mis en œuvre par ordinateur selon la revendication 2, dans lequel pendant l'exécution de l'ordre composé (24) de la fonction de micrologiciel critique pour le patient (10) du dispositif médical implantable (12), la seconde somme de contrôle (CRC_B) est écrasée par la première somme de contrôle (CRC_A), ladite première somme de contrôle (CRC_A) étant lue depuis un tampon de mémoire (26a) ou depuis une zone de code supplémentaire (26b) de la fonction de micrologiciel critique pour le patient (10) du dispositif médical implantable (12).

4. Procédé mis en œuvre par ordinateur selon la revendication 3, dans lequel après l'étape consistant à écraser la seconde somme de contrôle (CRC_B) par la première somme de contrôle (CRC_A), l'ordre composé (24) de la fonction de micrologiciel critique pour le patient (10) du dispositif médical implantable (12) est achevé.

5. Procédé mis en œuvre par ordinateur selon l'une quelconque des revendications précédentes, dans lequel la seconde somme de contrôle (CRC_B) est lue depuis un registre matériel en lecture seule du dispositif médical implantable (12), et dans lequel la seconde somme de contrôle (CRC_B) est un module de contrôle de redondance cyclique, ou XOR, ou un hachage cryptographique, en particulier MD5, SHA-1 ou SHA-2.

6. Procédé mis en œuvre par ordinateur selon l'une quelconque des revendications précédentes, dans lequel la fonction de micrologiciel critique pour le patient (10) du dispositif médical implantable (12) est exécutée en évaluant une interface utilisateur graphique d'un dispositif de programmation (30) ou d'une application fonctionnant sur un appareil mobile (32), en particulier un appareil de type smartphone ou tablette, le dispositif de programmation (30) ou appareil mobile (32) étant configuré pour communiquer sans fil avec le dispositif médical implantable (12).

7. Procédé mis en œuvre par ordinateur selon la revendication 6, dans lequel une procédure d'authentification d'utilisateur comprend une entrée de mot de passe ou une authentification à deux facteurs comprenant une entrée de mot de passe et une caractéristique de sécurité supplémentaire sur le dispositif de programmation (30) ou une interface web configurée pour commander le dispositif de programmation (30), et dans lequel la session utilisateur comprend un ID de session et un horodatage.

8. Procédé mis en œuvre par ordinateur selon l'une quelconque des revendications précédentes, dans lequel la somme de contrôle correcte (CRC_OK) associée à la fonction de micrologiciel critique pour le patient (10) du dispositif médical implantable (12) est calculée et comparée à la seconde somme de contrôle (CRC_B) au sein d'un intervalle de temps prédéfini, en particulier allant jusqu'à 500 ms, avant exécution de la fonction de micrologiciel critique pour le patient (10) du dispositif médical implantable (12).

9. Procédé mis en œuvre par ordinateur selon l'une quelconque des revendications précédentes, dans lequel la seconde somme de contrôle (CRC_B) est écrite au moment d'une initialisation usine du dispositif médical implantable (12) dans une cellule de mémoire prédéfinie, ladite cellule de mémoire étant accessible en exécutant un code de registre prédéfini.

10. Système (1) destiné à protéger une fonction de micrologiciel critique pour le patient (10) d'un dispositif médical implantable (12), en particulier d'un stimulateur cardiaque, d'un défibrillateur et/ou d'un neurostimulateur, contre une exécution non souhaitée, le système comprenant :
un dispositif médical implantable (12) et un dispositif de programmation (30), dans lequel le dispositif médical implantable (12) est configuré pour recevoir une requête (14) par le dispositif de programmation (30) pour exécution d'une fonction de micrologiciel critique pour le patient (10) du dispositif médical implantable (12), dans lequel le dispositif médical implantable (12) est configuré pour vérifier qu'un utilisateur associé à la requête (14) est autorisé à exécuter la fonction de micrologiciel critique pour le patient (10), dans lequel le dispositif médical implantable (12) est configuré pour lire une première somme de contrôle (CRC_A) depuis une première zone de mémoire (16) du dispositif médical implantable (12) ou pour fournir une première somme de contrôle (CRC_A) en tant que partie d'une zone de code (18) de la fonction de micrologiciel critique pour le patient (10) du dispositif médical implantable (12), dans lequel la première somme de contrôle (CRC_A) ne correspond pas à une somme de contrôle correcte (CRC_OK) associée à la fonction de micrologiciel critique pour le patient (10) du dispositif médical implantable (12), dans lequel le dispositif médical implantable (12) est configuré pour lire une seconde somme de contrôle (CRC_B) depuis une deuxième zone de mémoire (20) du dispositif médical implantable (12), dans lequel la seconde somme de contrôle (CRC_B) correspond à la somme de contrôle correcte (CRC_OK) associée à la fonction de micrologiciel critique pour le patient (10) du dispositif médical implantable (12), dans lequel le dispositif médical implantable (12) est configuré pour écrire la seconde somme de contrôle (CRC_B) dans une troisième zone de mémoire (22) du dispositif médical implantable (12) depuis laquelle une somme de contrôle est lue avant exécution de la fonction de micrologiciel critique pour le patient (10) du dispositif médical implantable (12), dans lequel le dispositif médical implantable (12) est configuré pour calculer la somme de contrôle correcte (CRC_OK) associée à la fonction de micrologiciel critique pour le patient (10) du dispositif médical implantable (12) et pour la comparer à la seconde somme de contrôle (CRC_B), et dans lequel le dispositif médical implantable (12) est configuré pour exécuter la fonction de micrologiciel critique pour le patient (10) du dispositif médical implantable (12) si la seconde somme de contrôle (CRC_B) et la somme de contrôle correcte (CRC_OK) correspondent.

11. Programme informatique avec un code de programme destiné à réaliser le procédé selon l'une quelconque des revendications 1 à 9 lorsque le programme informatique est exécuté sur un ordinateur.

12. Dispositif de transport de données lisible par ordinateur contenant un code de programme d'un programme informatique destiné à réaliser le procédé selon l'une quelconque des revendications 1 à 9 lorsque le programme informatique est exécuté sur un ordinateur.
